# EUROPÄISCHE PATENTSCHRIFT

(11) **EP 2 683 421 B1**
(45) Veröffentlichungstag und Bekanntmachung des Hinweises auf die Patenterteilung: **26.07.2017**
(21) Anmeldenummer: 12719929.7
(22) Anmeldetag: 14.02.2012
(51) Int. Cl.: A61L 27/54, A61L 29/16, A61L 31/16

(54) **ENDOPROTHESE MIT EINER WIRKSTOFFBESCHICHTUNG**
ENDOPROSTHESIS HAVING AN ACTIVE SUBSTANCE COATING
ENDOPROTHÈSE DOTÉE D'UN REVÊTEMENT DE SUBSTANCE ACTIVE

(30) Priorität: 11.03.2011 DE 102011014386
(43) Veröffentlichungstag der Anmeldung: 15.01.2014
(73) Patentinhaber: Hemoteq AG, 52146 Würselen (DE)
(72) Erfinder: HOFFMANN, Erika, 52249 Eschweiler (DE); HOFFMANN, Michael, 52249 Eschweiler (DE); HORRES, Roland, 52223 Stolberg (DE); ERDTMANN, Martin, 52428 Jülich (DE); HORBACH, Helmut, 52074 Aachen (DE)
(74) Vertreter: Arth, Hans-Lothar
(86) Internationale Anmeldenummer: PCT/DE2012/100035
(87) Internationale Veröffentlichungsnummer: WO 2012/122973

(56) Entgegenhaltungen:
- EP-A1- 1 243 259

## Beschreibung

Die vorliegende Erfindung betrifft Endoprothesen mit einer Wirkstoffbeschichtung enthaltend oder bestehend aus mindestens einem Antibiotikum und mindestens einer weiteren Substanz gemäß einer der weiter unten definierten Formeln (IVa), (IVb), (Va), oder (Vb).

Bei Endoprothesen handelt es sich um Implantate, welche dauerhaft oder über einen längeren Zeitraum im Körper verbleiben und den geschädigten Körperteil ganz oder teilweise ersetzen. Die Anforderungen an solche Ersatz- bzw. Hilfsteile sind sehr hoch, wie auch in Abhängigkeit von den Bedingungen am Implantationsort, höchst unterschiedlich. Man unterscheidet zwischen medizinischen, funktionellen, plastischen Endoprothesen, wie auch Zahnimplantaten. Als Implantate werden Herzschrittmacher, Stents und Gefäßprothesen, Implantate zur tiefen Hirnstimulation, sogenannte Hirnschrittmacher, wie sie z.B. bei Parkinson eingesetzt werden, Kunstherz und Portkatheter, orthopädische Implantate wie Gelenkersatz oder Materialen, die zur operativen Behandlung von Knochenbrüchen verwendet werden, Sehimplantate wie Augenlinsen, Retina, Glaskörper, Cornea, Zahnimplantate, Schädelrekonstruktionen, Knochenersatz, Penisprothesen, Schließmuskelprothesen, Cochleaimplantate, aber auch wieder entfernbare über einen begrenzten Zeitraum eingesetzte funktionsunterstützende Hilfsmittel wie Katheter, z.B. Blasenkatheter, Herzkatheter, Beatmungsschläuche, Venenkatheter, Kanülen aber auch rein als Wirkstoffdepot eingesetzte Implantate verstanden.

Endoprothesen werden aus biokompatiblen Materialen hergestellt, die je nach Einsatzgebiet in Material, und Aussehen sehr unterschiedlich sein können und z.B. aus Metall, Metalllegierungen, Kunststoff bzw. Polymeren wie z.B. PEAK (Polyaryletherketone), PEEK (Polyetheretherketon), PEK, PEEEK, PEEKEK und PEKK, Keramik aber auch aus deren Kombinationen bestehen. In Abhängigkeit von der Materialzusammensetzung und des Einsatzgebiets sind die Endoprothesen und Revisionsendoprothesen, deren Anteil an operativen Einsätzen nicht unwesentlich sind, biostabil, bioabbaubar, bioinert oder bioaktiv und haben eine raue oder glatte, mikroporöse oder makroporöse, hydrophile oder hydrophobe Oberfläche, sind bereits beschichtet oder in irgendeiner anderen Form vorbehandelt. Für die Beschichtung ist neben dem Material selbst auch die Form des zu beschichtenden Körpers von Bedeutung, da die geometrischen Strukturen unterschiedliche Beschichtungstechniken erfordern können. Hier lassen sich Verfahren wie Tauchbeschichtung, Sprühbeschichtung, Pipettierbeschichtung, Elektrospinning etc. als auch Kombinationen der möglichen Verfahren anführen, mit denen die optimale Vorgehensweise zu einer hochwertigen, den Anforderungen angepassten Beschichtung erreicht werden können und damit den Erfolg einer therapeutisch wirksamen Beschichtung positiv beeinflussen.

Die optimal wirksame Beschichtung einer Endoprothesenoberfläche sollte die vorgesehene Endoprothesenlebensrate bzw. mindestens die notwendige Verbleibdauer im Organismus erhöhen und eine Revision der Prothese verhindern können, die sich auch trotz optimaler Prothesenpassform und komplikationsloser Einsetzung notwendig werden kann. Die Adaption des Implantats an das umliegende Gewebe soll durch die Beschichtung erleichtert werden, einen komplikationslosen Heilungsprozess unterstützen und die Akzeptanz des Fremdkörpers im Organismus fördern und mögliche Störfaktoren, die den Heilungsprozess be- bzw. verhindern können, möglichst verhindern oder vermindern.

Beispielsweise sollen orthopädische Implantate wie z.B. eine im Knochenbereich eingesetzte Prothese wie z.B. die Hüftgelenkprothese oder Kniegelenkprothese eine starke unmittelbare Bindung mit dem Wirtsknochen eingehen. Diese Bindung lässt sich durch die Beschichtung der Oberfläche mit einer biologisch aktiven Schicht aus z.B. Hydroxylapatit oder anderen sinnvollen Calciumphosphatbeschichtungen verbessern, die ein wesentlicher mineralischer Knochenbestandteil sind. Ein Beispiel weiterer Entwicklungen im Bereich von derartigen Knochenimplantatsbeschichtungen ist unter dem Vermarktungsnamen Palacos R+G Beschichtung von Heraeus bekannt, die aus einer hochviskosen Knochenzementmischung und Gentamicinsulfat besteht. Doch auch hier gibt es immer wieder Probleme mit der Akzeptanz am Wirtsknochen und somit ist auch diese Beschichtung nur bedingt erfolgreich, da die Elution des Wirkstoffes aus der Matrix nicht dosisadaptiert verläuft und die wirkstofffreie Matrix als für den weiteren Heilungsprozess und für die langzeitliche Nutzungsbedingungen der Endoprothese unzureichend angenommen werden. Damit wird das angestrebte Ziel verfehlt, da, obwohl die Revisionsraten mit Palacos R+G rückgängig sind, die Ergebnisse immer noch nicht zufriedenstellen.

In anderen Bereichen, wie z.B. bei Augenlinsenimplantaten oder auf der Lumenseite von Speiseröhrengefäßstützen ist eine glatte, hydrophile und optimalerweise auch säurebeständige Oberfläche notwendig. Die Optimierung solcher Prothesen durch anwendungsorientierte Beschichtungen ist ein überaus weites Feld, dessen Entwicklung noch lange nicht abgeschlossen ist und immer wieder auf individuelle Schwierigkeiten stößt, die sich durch die Art der Erkrankung, Ort der Erkrankung, den vorgegebenen Bedingungen und natürlich auch nach der Verfassung des Patienten richten müssen.

Damit lässt sich kurz zusammenfassen, dass, obwohl es bereits eine Vielzahl von Beschichtungsvarianten für Endoprothesen gibt, Endoprothesen in allen Einsatzgebieten weiterhin Probleme zeigen, die die Lebensqualität des Patienten nicht verbessern können und zur Revision des Implantates führen. Die Ursachen für Komplikationen sind vielfältig und lassen sich grob in zwei Gruppen unterteilen:
1. Operationstechnik, Materialfehler, Designprobleme allgemeiner Art aber auch aufgrund individuell auftretender biologischer Diversifikationen, etc.
2. Allgemein ungünstige Adaptionsvorgänge unterschiedlicher Herkunft, Knochen- oder Knorpelabrieb, Entzündungsvorgänge, Keimbesiedlung, Reaktionen des Immunsystems auf den Fremdkörper.

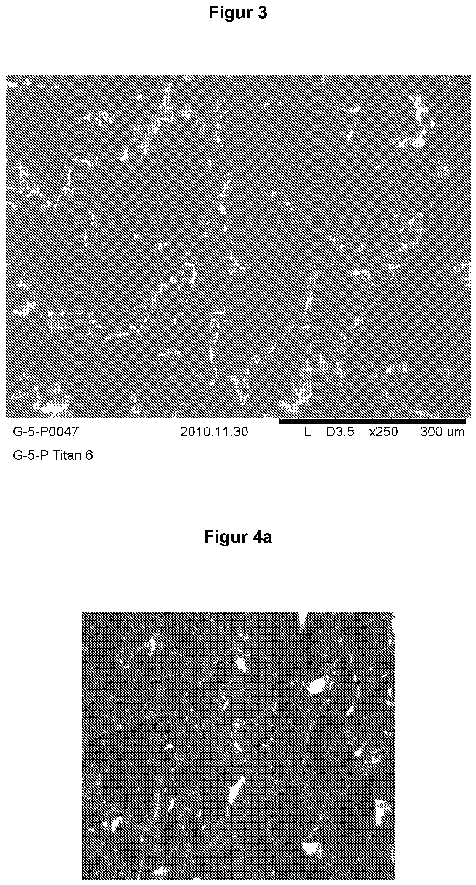

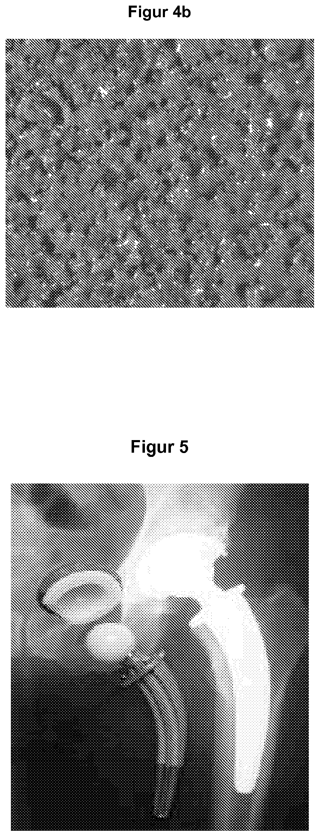

Die Beschichtung von Endoprothesen mit reinen Antibiotika ohne Additive oder Matrix zeigt keine Erfolge, da das Antibiotikum sich zum einen in kürzester Zeit auflöst und zum anderen mit der Substanz keine an der Endoprothese haftende Schicht darstellbar ist, da die Beschichtung pulverig, trocken, spröde und bröselig ist. Folglich lässt sich auch nicht nachvollziehen, wie viel des Antibiotikums den Bestimmungsort erreicht und wirksam sein kann. Unter diesen Bedingungen ist der therapeutische Effekt aufgrund fehlender Genauigkeit und Reproduzierbarkeit der abgegebenen Mindestmengen und schlussfolgernd die Effektivität der beschichteten Endoprothese in Frage gestellt. Diese Defizite können den unterschiedlichen, geforderten Bedingungen am Implantationsort nicht annähernd gerecht werden und keinesfalls für einen adäquaten optimalen und komplikationslosen Heilungsprozess garantieren. Figur 4a und b zeigen die spröden Oberflächen einer mit Gentamicinsulfat sprühbeschichteten Endoprothese. Figuren 4a und 4b zeigen anschaulich die nicht feste, brüchige, spröde Gentamicinsulfatbeschichtung, wo das Gentamicinsulfat nicht fest an der Oberfläche des Medizinproduktes haftet und daher die Beschichtung leicht abplatzt und insgesamt ungeeignet ist, um ein Medizinprodukt mit einer Beschichtung zu versehen, welche den Anforderungen an ein Medizinprodukt, dessen Lagerung, Sterilisation und regulatorischen Zulassungsanforderungen genügt. Die Patentanmeldung EP 1 243 259 A offenbart Implantate mit einer Beschichtung aus einem Antibiotikum-Polymer-Komposit. Die Beschichtung haftet fest auf den Oberflächen von Kunststoff- und Metallimplantaten und erlaubt eine kontinuierliche Freisetzung von Antibiotika über einen Zeitraum von mehreren Tagen bis zu Wochen.

Eine aus Gentamicinsulfat und der höherkettigen ungesättigten Fettsäure Palmitinsäure (C16:0) erhältliche Beschichtung von Endoprothesen zeigt leider keine kontrollierte in vitro-Freisetzung aus Gentamicinpalmitat und hohe Anfangselutionskonzentration ohne erkennbare Freisetzungsverzögerung. Zudem werden nur bis zu 58% des Gentamicinsulfats freigesetzt, womit auch eine unzureichende therapeutische Wirkung erreicht wird. Das Gentamicinsulfat bildet somit eine brüchige und spröde Beschichtung, welche bereits bei der Sterilisation und Verpackung des beschichteten Medizinproduktes abplatzt und zum anderen haften Bereiche der Beschichtung derartig fest auf der Implantatsoberfläche, dass aus diesen Bereichen fest kein Gentamicin freigesetzt wird, so dass letztendlich nur eine etwas über 50% liegende Freisetzung des Gentamicins erreicht wird. Daher ist die Gentamicinsulfatbeschichtung zur Aufbringung und Freisetzung des Antibiotikums Gentamicin von einer Oberfläche eines Medizinproduktes ungeeignet. Zudem ist neben der ungenügenden Freisetzung des Antibiotikums trotz bzw. gerade aufgrund der besseren Haftung des Antibiotikums auf der Endoprothese in Palmitatmatrix die Toxizität von Palmitinsäure als bedenklich einzustufen, da auch Palmitinsäure sich als cytotoxisch erweist und daher nicht als optimale Matrix für das Antibiotikum geeignet ist und als solches den weiteren Heilungsverlauf behindert, was sich ganz besonders im Bereich der Knochenimplantate bemerkbar macht, da sich der Abtransport bzw. Diffusion und Diffusionsgeschwindigkeit von Substanzen in die Umgebung im Vergleich zu einem Implantat in flüssigkeitsführender Umgebung wesentlich voneinander unterscheidet.

Aufgabe der vorliegenden Erfindung ist es, beschichtete Endoprothesen bereitzustellen, welche die bekannten Nachteile nach einer Implantation vermeiden bzw. merklich vermindern bzw. den Anspruch erhebt, die mindestens notwendigen Anforderungen, die an eine Endoprothese gestellt werden, um einen komplikationslosen Verlauf der Heilung und der unproblematischen langlebigen Nutzung zu garantieren, erfüllt. Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Es wurde gefunden, dass die genannten Nachteile, die sich nach einer Implantierung von Endoprothesen und insbesondere von knochenkontaktierenden Implantaten ergeben, durch eine Wirkstoffbeschichtung enthaltend oder bestehend aus mindestens einem Antibiotikum und mindestens einer Substanz der Formel (IVa), (IVb), (Va), oder (Vb) vermieden bzw. gemindert werden können. Es hat sich überraschenderweise gezeigt, dass die erfindungsgemäße Beschichtung bestehend aus mindestens einem Antibiotikum und mindestens einer Substanz der Formel (IVa), (IVb), (Va), oder (Vb) sich durch eine bevorzugte Elutionskinetik auszeichnet. Das Antibiotikum wird aus der erfindungsgemäßen Beschichtung schnell und vollständig freigesetzt. Zudem zeichnet sich die Beschichtung durch eine verringerte Sprödigkeit und Brüchigkeit und eine bessere Anhaftung an den Endoprothesen trotz vollständiger Freisetzung aus, so dass die Gefahr einer Ablösung der Beschichtung während des Transports, der Lagerung, der Sterilisation oder der Implantation stark verringert ist. Die Beschichtung enthält oder besteht aus aus einem Antibiotikum und Dexpanthenol oder Pantothensäure, bzw. Pantoinsäure oder Pantocain und/oder deren Derivate wie beispielsweise Formiate, Acetate, Propionate, Ethylester oder Ethylether. Besonders bevorzugt ist auch eine Beschichtung enthaltend oder bevorzugt bestehend aus mindestens einem Aminoglykosid-Antibiotikum mit Pantothensäure oder mit Dexpanthenol. Pantothensäure ((R)-N-(2,4-Dihydroxy-3,3-dimethyl-1-oxobutyl)-β-alanin) ist ein Vitamin aus der Gruppe der B-Vitamine (Vitamin B5), dass mit der Nahrung aufgenommen wird und spielt als Bestandteil der Acyltransferase Coenzym A eine wesentliche Rolle im Stoffwechsel. Man findet Pantothensäure vorrangig als Bestandteil von Haarpflegeprodukten und Produkten gegen Akne. Pantothensäure kann durch eine der folgenden Formeln dargestellt werden:

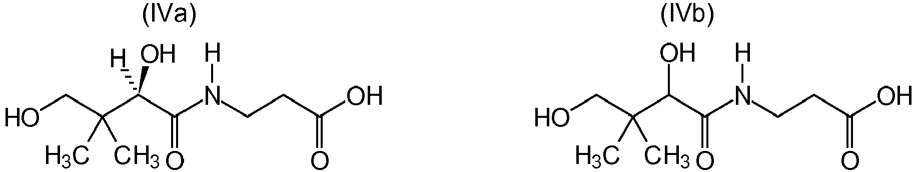

Dexpanthenol (auch bezeichnet als Pantothenol, Panthenol, D-Panthenol) mit dem systematischen Namen (+)-(R)-2,4-Dihydroxy-N-(3-hydroxypropyl)-3,3-dimethyl-butyramid und der folgenden Strukturformel 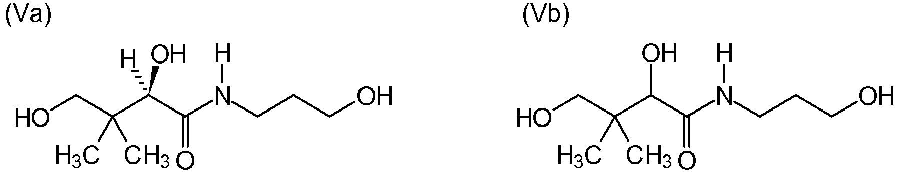 wird seit langem topisch als Mittel gegen Haut- und Schleimhauterkrankungen und in der Kosmetik verwendet, da es juckreizlindernde, entzündungshemmende, wundheilungsfördernde und zellneubildungsfördernde Eigenschaften hat und als Zusatz in Wasser-Öl-Emulsionen gut von der Haut aufgenommen wird, wobei es sich am Anwendungsort anreichert und aufgrund seiner hydrophilen Eigenschaften das Feuchthaltevermögen der Haut erhöht und somit neben den pflegenden Eigenschaften die Elastizität der Haut verbessert und die Neubildung der Hautzellen unterstützt, womit es zur Regeneration beiträgt. Zu den Dexpanthenol-enthaltenden Produkten gehören deshalb topisch anzuwendende Wundheilungspräparate wie Brand- und Wundgele, Wundsalben, Augen- und Nasensalben, Nasensprays, Venensalben, Mundgele, Salben zur Behandlung von Hämorrhoiden, Aknepräparate, Pflegecremes zur Anwendung bei trockener entzündlicher Haut. Es ist aber auch in Lutschtabletten bei Halsentzündungen und in Injektionslösungen zu finden.

Innerhalb des Organismus wird es als Bestandteil von Vitaminpräparaten in Form von Kapseln, Tabletten und Injektionslösungen und in Kontaktlinsenreinigungsprodukten verwendet. Dexpanthenol wird im Körper zur ebenfalls als Beschichtungsbestandteil bevorzugten Pantothensäure (Vitamin B5) umgewandelt und ist damit ebenso bedenkenlos als Beschichtung für Endoprothesen anwendbar.

Antibiotika, die in der erfindungsgemässen Beschichtung eingesetzt werden können, umfassen unter anderem Penicillin, Penicillin G und V, Amikacin, Amoxicillin, Ampicillin, Bacampicillin, Carbenicillin, Indanyl Pivmecillinam, Oxacillin, Flucloxacillin, Aminopenicilline, Aminocumarine, Azithromycin, Mezlocillin, Piperacillin, Azlocillin, Temocillin, Ticarcillin, Amoxicillin, Clavulansäure, Ampicillin, Sulbactam, Piperacillin, Tazobactam, Sulbactam, Cephalosporine, Cefazolin, Cefamandol, Cefotiam, Cefuroxim, Cefmenoxim, Cefodizim, Cefoperazon, Cefotaxim, Ceftazidim, Cefsulodin, Ceftriaxon, Cefepim, Cefpirom, Cefoxitin, Cefotetan, Cefaclor, Cefadroxil, Cefalexin, Cefuroxim Axetil, Cefixim, Cefpodoxim, Ceftibuten, Chlorhexidin, Imipenem, Gramidicin, Kanimycin, Cethromycin, Narbomycin, Telithromycin, Lincomycin, Meropenem, Ertapenem, Doripenem, Aztreonam, Josamycin,Erythromycin, Roxithromycin, Clarithromycin, Spiramycin, Polymyxin B, Azithromycin, Telithromycin, Quinopristin, Dalfopristin, Clindamycin, Tetracyclin, Oxytetracyclin, Doxycyclin, Minocyclin, Trimethoprim, Tyrothricin, Sulfamethoxazol, Sulfametrol, Nitrofurantoin, Lomefloxacin, Norfloxacin, Ciprofloxacin, Ofloxacin, Fleroxacin, Levofloxacin, Ofloxacin, Enoxacin, Fosmidomycin, Sparfloxacin, Methicillin, Tinidazol, Moxifloxacin, Vancomycin, Teicoplanin, Linezolid, Daptomycin, Rifampicin, Fusidinsäure, Fosfomycin, Trometamol, Chloramphenicol, Metronidazol, Colistin, Mupirocin, Bacitracin, Neomycin, Netilmycin, Tigecyclin, Sulfasalazin, Sulfadiazin, Sulfadoxin, Fluconazol, Itraconazol, Voriconazol, Posaconazol, Pyrimethamin, Trimethoprim, Amphotericin B, 5- Flucytosin, Caspofungin und/oder Anidulafungin.

Besonders bevorzugt sind Hemmer der Zellwandsynthese wie Imipenem, Meropenem, Ertapenem, Aztreonam, Pencilline, Aminpenicilline, Acylaminopenicilline, Isoxazolylpenicilline, Cephalosporine, Sultamicillin, Fosfomycin, Glycopeptide wie Vancomycin und Teicoplanin, Polypetide wie Bacitracin, Colistin, Gramicidin, Polymyxin B, Tyrothricin und der Proteinsynthese am Ribosom wie die Antibiotika aus der Gruppe der Aminoglykoside auch Aminoglykosid-Antibiotika genannt. Die Gruppe der Aminoglykoside, die für eine erfindungsgemäße Beschichtung besonders bevorzugt sind umfasst bzw. besteht aus: Streptomycin, Neomycin, Framycetin, Paromomycin, Ribostamycin, Kanamycin, Amikacin, Arbekacin, Bekanamycin, Dibekacin, Tobramycin, Spectinomycin, Hygromycin B, Paromomycinsulfat, Gentamicin, Netilmicin, Sisomicin, Isepamicin, Verdamicin, Astromicin, Apramycin, Ansamycine wie z.B. Rifampicin, Geneticin. Ein besonders bevorzugtes Aminoglykosid ist Gentamicin (Typgemisch) sowie alle Einzelverbindungen, die zur Gruppe der Gentamicine gehören, z.B. Gentamicin B (Betamicin) oder Gentamicin C₁. Der pharmazeutisch verwendete Arzneistoff setzt sich aus mehreren Einzelverbindungen der Stoffgruppe der Gentamicine zusammen, enthalten sind fast ausschließlich Gentamicine vom Typ C. Sofern im Folgenden nichts anders vermerkt ist handelt es sich bei dem Begriff Gentamicin immer um dieses Typgemisch.

Aminoglykoside gehören zu der Gruppe der Oligosaccharid-Antibiotika, mit Kombinationen aus Aminozucker- und Cyclohexan-Bausteinen. Die Ausscheidung erfolgt mit einer kurzen Halbwertszeit von etwa zwei Stunden vorwiegend über die Nieren.

Weitere bevorzugte Antibiotika sind: Daptomycin, Tigecyclin, Chloramphenicol, Doxycycline, Monocyclin, Tetracyclin, Oxytetracyclin, Azithromycin, Clarithromycin, Erythromycin, Roxithromycin, Dalfopristin, Quinupristin, Clindamycin, Lincomycin, Telithromycin, Narbomycin, Cethomycin und Fusidinsäure.

Es ist auch möglich, dass Kombinationen aus mindestens zwei der genannten Antibiotika in einer erfindungsgemäßen Beschichtung enthalten sind. Diese können gemeinsam in einer Schicht der Beschichtung vorliegen oder in getrennten Schichten übereinander angeordnet sein, oder in verschiedenen Bereichen der Prothese aufgebracht werden.

Die Wahl des Antibiotikums und dessen Konzentration richtet sich nach den üblicherweise im Zusammenhang mit der beschichteten Endoprothese am häufigsten auftretenden Infektion und eventuell auch den bekannten Unverträglichkeiten des zu behandelnden Patienten.

Die verwendeten Antibiotika können biologischer, semisynthetischer und synthetischer Herkunft sein.

Mit Hilfe einer erfindungsgemäßen Wirkstoffbeschichtung lassen sich Beschwerden, die nach einer Implantation auftreten können, z.B. die Revisionsrate also die frühzeitige Entfernung einer Endoprothese, wie z.B. einer Hüftgelenksprothese, merklich vermindern. Vor allem durch grampositive Erreger und gramnegative Enterobakterien und Nichtenterobakterien verursachte Infektionen sollen damit im Idealfall verhindert aber mindestens auf eine unbedenkliche Form minimiert werden, so dass der Organismus die wenigen eventuell verbleibenden Keime selbst erfolgreich bekämpfen kann.

Es ist bevorzugt, wenn die gesamte Endoprothese gleichmäßig beschichtet ist. Ferner ist es bevorzugt, wenn eine gleichmäßige Verteilung des mindestens einen Antibiotikums und der Substanz der Formel (IVa), (IVb), (Va), oder (Vb) auf der Endoprothese vorliegt.

In der Regel ist eine vollflächige Wirkstoffbeschichtung der Endoprothese vorteilhaft, d.h. die gesamte Oberfläche der Endoprothese wird mit einer Beschichtung versehen. Die Beschichtung der Endoprothese kann ferner noch dahingehend ausgestaltet werden, dass die Beschichtung mit dem Gemisch aus dem Antibiotikum und der Substanz der Formel (IVa), (IVb), (Va), oder (Vb) nicht gleichmäßig erfolgt, sondern man einen Gradienten verwendet, d.h. ein Konzentrationsgefälle auf der Endoprothese erzeugt wird. So kann beispielsweise in der Mitte oder an bestimmten Bereichen der Endoprothese eine größere Konzentration von Antibiotikum und der Substanz der Formel (IVa), (IVb), (Va), oder (Vb) aufgebracht werden, als auf dem Rest der Endoprothese.

Zudem kann auch nur an einer Stelle oder einem Teil der Endoprothese eine höhere Konzentration des Antibiotikums und der Substanz der Formel (IVa), (IVb), (Va), oder (Vb) aufgebracht werden als auf der restlichen Oberfläche. Beliebige Variationen sind hier denkbar.

Der Begriff "Beschichtung" oder "Wirkstoffbeschichtung" soll nicht nur eine Beschichtung der Oberfläche sondern auch eine Befüllung oder Beschichtung von Falten, Kavitäten, Poren, Mikronadeln oder anderen befüllbaren Räumen auf oder zwischen oder in der Endoprothese umfassen.

Die Oberfläche kann zusätzlich mit einer hämokompatiblen Schicht als Basisbeschichtung versehen werden, die durch kovalente Immobilisierung von semisynthetischen Heparinderivaten, wie z.B. desulfatiertem, reacetyliertem Heparin, oder Chitosanderivaten, wie z.B. N-carboxymethyliertes, partiell N-acetyliertes Chitosan, aufgebracht wird.

Alle Endoprothesen lassen sich mit solch einer Beschichtung versehen. Es ist auch möglich, die Endoprothesen nur teilweise zu beschichten.

Zu der Beschichtung aus dem mindestens einen Antibiotikum und der Substanz mit der Formel (IVa), (IVb), (Va), oder (Vb) können ein oder mehrere weitere Wirkstoffe, bevorzugt eine antiphlogistische, antineoplastische, antiangiogene, antiproliferative oder immunsuppressive Substanz zugemischt werden.

Anwendbare antiphlogistische, antineoplastische, antiangiogene, antiproliferative oder immunsuppressive Substanzen sind unter anderem Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid Estramustin, Melphalan, Betulinsäure, Camptothecin, Lapachol, ß-Lapachon, Podophyllotoxin, Betulin, Tropfosfamid, Podophyllsäure-2-ethylhydrazid, Ifosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Mofebutazon, Acemetacin, Diclofenac, Lonazolac Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense Selectin (Cytokinantagonist) CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, Folsäure und Derivate, Vitamine der B-Reihe, Vitamin D-Derivate wie z.B. Calcipotriol und Tacalcitol, Thymosin □-1, Fumarsäure und ihre Derivate wie z.B. Dimethylfumarat, IL-1ß-Inhibitor, Colchicin, NO-Donoren wie Pentaerythrityltetranitrat und Syndnoeimine, S-Nitrosoderivate, Tamoxifen, Staurosporin, ß-Estradiol, α-Estradiol, Estron, Estriol, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate (6-α-Hydroxy-Paclitaxel, Baccatin, Taxotere u.a.), synthetisch hergestellte als auch aus nativen Quellen gewonnene macrocyclische Oligomere des Kohlensuboxids (MCS) und seine Derivate, Molgramostim (rhuGM-CSF), Peginterferon α-2b, Lanograstim (r-HuG-CSF), Filgrastim, Macrogol, Dacarbazin, Basiliximab, Daclizumab, Ellipticin, D-24851 (Calbiochem), Colcemid, Cytochalasin A-E, Indanocine, Nocadazole, S 100 Protein, PI-88, Melanocyte Stimulating Hormon (α-MSH), Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1 genannt. Positiven Einfluß auf die postoperative Phase haben auch Antithrombotika wie Argatroban, Aspirin, Abciximab, synthetisches Antithrombin, Bivalirudin, Coumadin, Enoxoparin, Hemoparin® (desulfatiertes und N-reacetyliertes Heparin), Gewebe-Plasminogen-Aktivator, GpIIb/IIIa-Plättchenmembranrezeptor, Faktor Xa-Inhibitor, aktiviertes Protein C, Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Caspaseinhibitoren, Apoptoseinhibitoren, Apoptoseregulatoren wie p65, NF-kB und Bcl-xL-Antisense-Oligonukleotiden und Prostacyclin, Vapiprost, α-,ß- und γ-Interferon, Histaminantagonisten, Serotoninblocker, Halofuginon, Nifedipin, Tocopherol, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron. Weitere Wirkstoffe sind Steroide (Hydrocortison, Betamethason, Dexamethason), nichtsteroidale Substanzen (NSAIDS) wie Fenoporfen, Ibuprofen, Indomethacin, Naproxen, Phenylbutazon und andere. Antivirale Agentien wie Acyclovir, Ganciclovir und Zidovudin sind ebenfalls einsetzbar. Verschiedene Antimykotika finden Anwendung in diesem Bereich. Beispiele sind Clotrimazol, Flucytosin, Griseofulvin, Ketoconazol, Miconazol, Nystatin, Terbinafin. Antiprozoale Agentien wie Chloroquin, Mefloquin, Quinin sind gleichermassen wirksame Agentien, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3, Tubeimosid, Bruceanole A,B,C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, 1,11-Dimethoxycanthin-6-on, 1-Hydroxy-11-Methoxycanthin-6-on, Scopolectin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-4,16-dien-3,20-dion, Bilobol, Ginkgol, Ginkgolsäure, Helenalin, Indicin, Indicin-N-oxid, Lasiocarpin, Inotodiol, Glykosid 1 a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-AII, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychno-pentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B, weitere natürliche Terpenoide wie Hippocaesculin, 14-Dehydroagrostistachin, Agroskerin, Agrostistachin, 17-Hydroxyagrostistachin, Ovatodiolide, 4,7-Oxy-cycloanisomelsäure, Yadanzioside N und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin. Diese Wirkstoffe werden einzeln oder kombiniert in gleicher oder unterschiedlicher Konzentration eingesetzt. Desweiteren kann eine zusätzliche Wirkstoffschicht, enthaltend eine antiphlogistische, antineoplastische, antiangiogene, antiproliferative und immunsuppressive Substanz ober oder unterhalb der Schicht der erfindungsgemäßen Verbindung liegen. Diese zusätzliche Wirkstoffschicht kann aus reinem Wirkstoff bestehen oder der zusätzliche Wirkstoff kann in eine polymere Beschichtung eingelagert sein. Entsprechende Polymere werden weiter unten aufgezählt. Die Wahl des Wirkstoffes und dessen Konzentration richtet sich nach dem individuellen Krankheitsbild und den daraus hervorgehenden Erfordernissen, die Genesung eines Patienten fördern zu können.

Je nach Bedarf kann die erfindungsgemäße Beschichtung auch auf einer vorhandenen unteren Lage (base layer) aufgebracht werden. Hierbei handelt es sich bevorzugt um eine Basisbeschichtung aus bioabbaubaren oder biostabilen Polymeren oder aus Calciumphosphat (z.B. Hydroxylapatit) oder aus keramischen Werkstoffen. Derartige Materialien können der erfindungsgemäßen Beschichtung auch beigemischt sein oder als Toplayer über einer erfindungsgemäßen Beschichtung aufgebracht werden, wobei auch in einer solchen Basisbeschichtung oder Toplayer ein Antibiotikum enthalten, bevorzugt dispergiert, sein kann.

Weitere Variationsmöglichkeiten für die Beschichtung bestehen in der kovalenten Anbindung eines Antibiotikums an das Endoprothesenmaterial, um eine permanente antibakterielle Oberfläche zu gestalten. In einem zweiten Schritt wird eine Substanz der Formel (IVa), (IVb), (Va), oder (Vb) aufgebracht. Diese Schicht kann mindestens ein weiteres Antibiotikum enthalten. Hierbei kann es sich um das Antibiotikum handeln, welches kovalent an die Endoprothesenoberfläche gebunden ist oder es handelt sich um ein zweites Antiobiotikum. Hierbei sind die Kombinationen von Antibiotika bzw. Antibiotikagemischen mit weiteren Wirkstoff(en) individuell anzupassen und folglich im Interesse des Patienten umzusetzen.

Ein bioabbaubares Polymer, welches in einer Basisbeschichtung, einem Toplayer oder der zusätzlichen Wirkstoffschicht enthalten sein kann, kann ausgewählt werden aus der Gruppe umfassend oder bestehend aus Polyvalerolactone, Poly-ε-Decalactone, Polylactonsäure, Polyglycolsäure Polylactide, Polyglycolide, Copolymere der Polylactide und Polyglycolide, Poly ε caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-p-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Polybutylenterephtalat. Polypivotolactone, Polyglycolsäuretrimethylcarbonate, Polycaprolactonglycolide, Poly-g-ethylglutamat, Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäureresten im Rückgrat, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen, Collagen-N-Hydroxysuccinimid, Lipide, Phospholipide, Modifikationen und Copolymere und/oder Mischungen der vorgenannten Substanzen.

Ein bioabbaubares Polymer, welches in einer Basisbeschichtung, einem Toplayer oder der zusätzlichen Wirkstoffschicht enthalten sein kann, kann ausgewählt werden aus der Gruppe umfassend oder bestehend aus Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polyvinylhalogene und Copolymere, Celluloseether, Cellulosetriacetate. Chitosan und Copolymere und/oder Mischungen derselben.

Erfindungsgemäß beschichtete Endoprothesen lassen sich durch ein Verfahren zur, Beschichtung herstellen, dem folgendes Prinzip zugrunde liegt:
a. Bereitstellen einer unbeschichteten Endoprothese oder einer Endoprothese, die mit einer Basisbeschichtung versehen ist und
b. im Wesentlichen vollständiges Überziehen der Oberfläche mit einer Beschichtungslösung aus mindestens einem Antibiotikum und mindestens einer Substanz der Formel (IVa), (IVb), (Va), oder (Vb)

Nach einer erfindungsgemäßen Beschichtung der Endoprothese mit einer Beschichtungslösung, enthaltend zumindest ein Antibiotikum und eine Verbindung der Formel (IVa), (IVb), (Va), oder (Vb), erhält man nach Verdunsten oder Entfernen des Lösungsmittels eine Antibiotikum-abgebende, entzündungshemmende und keimabtötende Formulierung, die die Verwendbarkeit hydrophiler Wirkstoffe auch auf hydrophobe Systeme erweitert.

Beschichtet werden die mit Knochen oder Gewebe in Kontakt kommenden Oberflächen der Endoprothese, bevorzugt des knochenkontaktierenden Implantats. Korrespondierende Oberflächen des Implantats, d.h. Oberflächen des Implantats, welche mit anderen Oberflächen des Implantats in Kontakt kommen oder kommen können, bedürfen keiner erfindungsgemäßen Beschichtung. Der Begriff "korrespondierende Oberfläche" ist dem Fachmann bekannt. Ein Beispiel für korrespondierende Oberflächen ist die Oberfläche der Tibiakomponente eines künstlichen Zwischenwirbelimplantats, welche mit dem Onlay in Kontakt kommen kann.

In der Regel wird der Beschichtungsvorgang einmal oder zweimal oder dreimal wiederholt, wobei dies jedoch nicht zwingend ist. Auch ein einmaliger Beschichtungsvorgang kann ausreichen, um die erforderliche Menge an Antibiotikum und der Substanz der Formel (IVa), (IVb), (Va), oder (Vb) auf die Endoprothese aufzutragen.

Die Beschichtung kann auch aktiv durch Erwärmung oder Anlegen von Vakuum oder im Gasstrom getrocknet werden. Unter dem Begriff Lösung, wie hierin verwendet, wird auch eine Emulsion oder eine Dispersion verstanden und beschränkt sich nicht nur auf klare homogene Lösungen.

Als Lösungsmittel für das Gemisch aus Antibiotikum und Substanz der Formel (IVa), (IVb), (Va), oder (Vb) und deren Derivaten kann Chloroform, Ethanol, Methanol, Tetrahydrofuran, Hexan, Aceton, Methylacetat, Essigsäureethylester, Methylenchlorid, DMSO oder ihre Gemische untereinander oder mit Wasser verwendet werden.

Es ist auch möglich, einen weiteren nicht polymeren Hilfsstoff als Matrix in die Mischung aus Antibiotikum und Substanz der Formel (IVa), (IVb), (Va), oder (Vb) zu geben. Es eignen sich beispielsweise Kontrastmittel oder Kontrastmittelanaloga sowie biologisch verträgliche organische Stoffe, die die Beschichtungseigenschaften verbessern. Es ist jedoch bevorzugt, wenn die erfindungsgemäße Beschichtung bzw. die Schicht aus mindestens einem Antibiotikum und mindestens einer Substanz der Formel (IVa), (IVb), (Va), oder (Vb) frei von Polymeren ist.

Die Beschichtung ist im Sprüh-, Tauch-, Pinsel-, Spritzen-, Schlepp-, Fadenschlepp-, Roll- und/oder Pipettierverfahren durchführbar und somit aus verfahrenstechnischer Sicht universell verwendbar, d.h. auf beliebig geformten Oberflächen anwendbar.

Unter dem Begriff "Beschichtungslösung" wie hierin verwendet, wird die Mischung aus der Verbindung von mindestens einem antibiotischen Wirkstoff mit mindestens einer Substanz der Formel (IVa), (IVb), (Va), oder (Vb) und und/oder dessen Derivate und einem Lösungsmittel oder Lösungsmittelgemisch und/oder weiterem Hilfsstoff verstanden, also eine tatsächliche Lösung, Dispersion, Suspension oder Emulsion. Der Begriff "Lösung" soll ferner verdeutlichen, dass es sich um ein flüssiges Gemisch handelt, wobei die Dichte des flüssigen Gemisches stark variieren kann.

Mit dem Begriff "Gemisch" oder "Mischung" ist die Kombination der mindestens zwei Stoffe gemeint, die in der Beschichtungslösung vorliegen. Bei der Mischung zweier Stoffe muss kein neuer Stoff entstehen. Die Definition des Begriffes "Gemisch" wie hierin verwendet, schließt aber auch die Bildung neuer Stoffe aus der Kombination mindestens zweier Stoffe ein. Damit sind auch ausdrücklich chemische Reaktionen zwischen den mindestens zwei Stoffen eingeschlossen. Bei dem Gemisch kann es sich um ein Gemenge, eine Legierung, ein Polymer, ein Co-Polymer, ein Komposit, einen Schwamm, einen Schaum, eine Lösung, eine Suspension, eine Emulsion, oder eine Dispersion handeln. Insbesondere kann es sich um homogene Gemische bestehend aus einer Phase und auch heterogene Gemische mit zwei oder mehr Phasen handeln. Die Definition des Begriffes "Gemisch" oder "Mischung" schließt auch ausdrücklich mit ein, dass sich die Stoffe in dem Gemisch möglicherweise nicht mehr in die Ausgangsstoffe auftrennen lassen und es ist auch möglich, dass die Stoffe ihre ursprünglichen Eigenschaften verlieren bzw. neue erhalten. Es ist möglich, dass die Stoffe in dem Gemisch über ionische, kovalente, van der Waals oder Wasserstoffbrückenbindungen miteinander verbunden sind.

In der Regel wird eine Menge von 1 µg bis 500 µg Antibiotikum pro cm² Oberfläche der zu beschichtenden Endoprothese, bevorzugt eine Menge von 10 µg bis 200 µg Antibiotikum pro cm² Oberfläche und besonders bevorzugt eine Menge von 20 µg bis 100 µg Antibiotikum pro cm² Oberfläche aufgetragen. Pro Endoprothese werden vorzugsweise 0,5 bis 1000 mg Antibiotikum und insbesondere bevorzugt werden pro Endoprothese 3 mg bis 200 mg auf die Endoprothese aufgetragen. Dies variiert jedoch stark je nach Größe der zu beschichtenden Endoprothese, der Art der zu beschichtenden Endoprothese, der Oberflächenbeschaffenheit und des verwendeten Antibiotikums. Das Verhältnis von dem mindestens einen Antibiotikum zu der mindestens einen Substanz der Formel (IVa), (IVb), (Va), oder (Vb) liegt bevorzugt zwischen 3 : 1 und 1: 10, insbesondere bevorzugt zwischen 1:1 und 1:5.

Bei Endoprothesen handelt es sich im Allgemeinen um Implantate, welche dauerhaft im Körper verbleiben und den geschädigten Körperteil ganz oder teilweise ersetzen. Der Begriff "Endoprothese", wie hierin verwendet, umfasst aber auch zusätzlich Prothesen und Implantate, die nicht dauerhaft aber dennoch über einen längeren Zeitraum (mindestens im Bereich von mehreren Tagen) im Körper verbleiben. Eine beispielhafte und nicht abschließende Liste von Endoprothesen im Sinne dieser Erfindung umfasst damit Zahnimplantate, Herzschrittmacher, Stents, Gefäßprothesen, Hirnschrittmacher, Kunstherzen, Portkatheter, orthopädische Implantate, Augenimplantate wie Sehimplantate, Retina, Glaskörper oder Cornea, Schädelrekonstruktionen, Knochenersatz, Penisprothesen, Schließmuskelprothesen, Cochleaimplantate, Katheter, Blasenkatheter, Beatmungsschläuche, Venenkatheter und Kanülen. Besonders bevorzugt sind orthopädische Implantate, die im Bereich des Knochengerüsts eingesetzt werden.

Als Beispiele solcher orthopädischer Implantate können genannt werden: Wirbelsäulenimplantate, Hüftgelenkimplantate, Hüftpfannen, Schultergelenkimplantate, Ellenbogengelenksimplantate, Fingergelenkimplantate, Fußgelenkimplantate, Zehgelenkimplantate, Kniegelenkimplantate, Sprunggelenkimplantate, Handgelenkimplantate oder generell Gelenkimplantate, Implantate für die Fusion von Knochen, Radiuskopf-Implantate, Pedikelschrauben, Verankerungsstifte von Implantaten oder für Implantate, Implantate für die Schädeldecke, Korrekturkeile, Winkelimplantate, Knochenkeile, Knochenschrauben, Zwischenwirbelimplantate wie z.B. Cages oder künstliche Bandscheiben oder Dornfortsatzspreizer, Knochenballons für die Kyphoplastie, Implantate für Winkelosteotomien (Tibiakopf), Mittelfußoperationen, Rückfußoperationen oder generell Implantate, welche Knochen verbinden oder in einen Knochen zumindest teilweise eingeführt werden. Ein besonders bevorzugtes orthopädisches Implantat ist ein Gelenksimplantat, insbesondere ein Hüftgelenksimplantat. Ein Hüftgelenksimplantat kann sowohl eine Kopfprothese als auch eine Schaftprothese (Stielprothese oder Femurschaft) und Hüftpfannenprothese sein.

Die vorgenannten orthopädischen Implantate sind in der Regel vollständig aus einem harten Material, insbesondere einem Metall oder einer Metalllegierung wie beispielsweise Titan, Zirkonium, oxidiertem Zirkonium, Hafnium, Platin, Rhodium, Niobium, medizinischem Edelstahl, CoCr-Stahl (Cobalt-Crom), Tantal und können aber auch aus faserverstärkten Kunststoffen (Glas- / Carbonfasern mit entsprechender Matrix), PEEK [Poly(etheretherketone)] oder Polymerwerkstoffen allgemein bestehen. Bei den Metalllegierungen können ferner Metalle wie Aluminium, medizinischer Stahl und/oder Gold zugesetzt werden.

Endoprothesen werden allgemein aus biokompatiblen Materialen hergestellt, die je nach Einsatzgebiet sehr unterschiedlich sein können. Die Oberfläche der zu beschichtenden Endoprothesen kann hydrophil oder hydrophob, rau oder glatt sein auch mikroporöse oder makroporöse sowie anderweitig strukturierte Oberflächen sind geeignet. Es ist auch möglich, dass die Endoprothesen vor Aufbringung der erfindungsgemäßen Beschichtung bereits beschichtet (mit einer Basisbeschichtung) oder in irgendeiner anderen Form vorbehandelt sind.

Die vorgenannten Implantate können auch keramsiche Beschichtungen zur Härtung aufweisen, worauf die erfindungsgemäßen Beschichtungen aufgebracht werden.

Weiterhin wurde gefunden, dass ein Beschichtungsverfahren der folgenden Art die gestellte Aufgabe besonders gut löst.

Dieses Verfahren zur Beschichtung einer Endoprothese umfasst die folgenden Schritte:
a) Bereitstellung einer unbeschichteten oder beschichteten Endoprothese,
b) Bereitstellen einer Beschichtungslösung enthaltend mindestens ein Antibiotikum und eine Substanz der Formel (IVa), (IVb), (Va), oder (Vb) in mindestens einem Lösungsmittel
c) Aufbringen der Beschichtungslösung mittels Sprühen, Tauchen, Streichen, Pinseln, Pipettieren, Gasphasenabscheidung oder Spritzen.

Es ist bevorzugt, wenn das mindestens eine Antibiotikum als Salz der mindestens einen Substanz der Formel (IVa), (IVb), (Va), oder (Vb) in der Beschichtung vorliegt, bzw. wenn in der Beschichtungslösung das mindestens eine Antibiotikum und die mindestens eine Substanz der Formel (IVa), (IVb), (Va), oder (Vb) als Anionen und Kationen vorliegen. Insbesondere bevorzugt ist, wenn ein Antibiotikum-Panthothenat zur Beschichtung der Endoprothese eingesetzt wird.

Hierzu ist ein bevorzugter, erster Verfahrensschritt zur Beschichtung der Endoprothese die Herstellung eines Salzes aus dem mindestens einen Antibiotikum und der mindestens einen Substanz der Formel (IVa), (IVb), (Va), oder (Vb), besonders bevorzugt durch Umsalzung. Eine Lösung dieses Salzes dient dann, eventuell mit weiteren Hilfsstoffen, als Beschichtungslösung.

### Figurenbeschreibung

**Figur 1** zeigt die Abhängigkeit der Oberflächenbelegung von Gentamicin-penta-pantothenat auf aufgerauten Titan-Prüfkörpern bei steigender Ausziehgeschwindigkeit.
**Figur 2** zeigt die Abhängigkeit der Oberflächenbelegung von Gentamicin-penta-pantothenat auf aufgerauten Titan-Prüfkörpern durch Wiederholen des Tauchvorgangs.
**Figur 3** zeigt eine REM-Aufnahme bei 250-facher Vergrößerung nach Sprühbeschichtung mit ethanolischen Gentamicin-penta-pantothenat Lösung. Die Beschichtung ist nicht pulverig, nicht spröde und brüchig, sondern hält zuverlässig auf der Endoprothesenoberfläche.
**Figur 4a** Mittels Pipettierverfahren auf vorgeheizten Titanscheiben mit mikroporöser Titanbeschichtung aufgetragene und anschliessend getrocknete Gentamicinsulfatbeschichtung (3, 1 mg Gentamicinsulfat/cm²) aus Aceton/Wasser (v/v 1:4) (200fache Vergrösserung). Die Beschichtung mit dem reinen Antibiotikum ist spröde, pulverig und bricht leicht ab und ist daher für ein zulassungspflichtiges Medizinprodukt ungeeignet.
**Figur 4b** Mittels Sprühverfahren auf vorgeheizten Titanscheiben mit mikroporöser Titanbeschichtung aufgetragene und anschliessend getrocknete Gentamicinsulfatbeschichtung (4,7 mg Gentamicinsulfat/cm²) aus Aceton/Wasser (v/v 1:3) Die Beschichtung wurde in 30 Einzelsprühschritten mit Zwischentrocknung aufgebracht (200fache Vergrösserung). Auch mit dem Sprühferfahren lässt sich die reine Antibiotikumbeschichtung nicht verbessern und ist spröde, pulverig und bricht leicht ab, womit die tatsächlich wirksame Dosis nicht verifiziert werden kann und der therapeutische Effekt in Frage gestellt ist.
**Figur 5** Antiinfektiv beschichtete Hüftgelenksprothese und Demonstration der Platzierung mittels Röntgenaufnahme

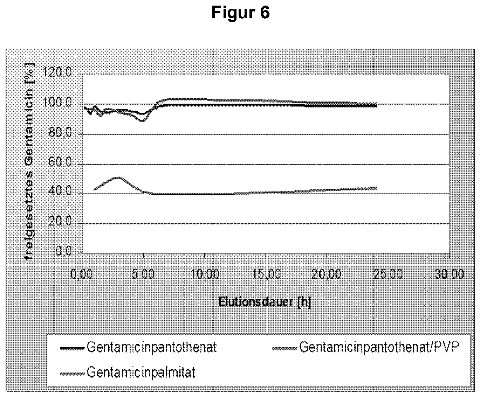

**Figur 6** In Vitro-Versuche zeigen, dass es keine kontrollierte in vitro-Freisetzung aus Gentamicinpalmitat (innerhalb von 11 Tagen) gibt, sondern vielmehr hohe Anfangskonzentration ohne erkennbare Freisetzungsverzögerung, wobei im Vergleich zum Gentamicinpantothenat als auch Gentamicinpantothenat/PVP mit 100% Freisetzung nur 58% des Gentamicins freigesetzt werden

### Beispiele

Von den folgenden Beispielen beziehen sich die Beispiele 14-18 und 40-45 auf die Erfindung, während die Beispiele 1-13 und 19-39 nicht erfindungsgemäß sind.

### 1 :Herstellung von Gentamicin-penta- dodecylsulfat

4,6 g Natriumdodecylsulfat wurden in 37,5 ml dest. Wasser gelöst und mit einer zweiten Lösung aus 2,5 g Gentamicinsulfat in 37,5 ml dest. Wasser vereinigt. Die Lösung wird mit 50 ml dest. Wasser verdünnt, wobei sich eine Emulsion bildet, aus der sich dann Ausflockungen absetzen. Der sich abgesetzte Feststoff wird abfiltriert und getrocknet.

### Beispiel 2 : Herstellung von Gentamicin-penta-pantothenat

2 g Gentamicinsulfat und 3,3 g Calciumdipantothenat werden in je 35 ml VE-Wasser gelöst und beide Lösungen mit weiteren 35 ml Wasser vereinigt. Die Lösung wurde filtriert und im Rotationsverdampfer bei 50° C bis zur Trocknung einrotiert und der ausgefallene Feststoff in Methanol aufgenommen. Die methanolische Suspension wurde anschließend zentrifugiert und das Sediment über Nacht nachgetrocknet.

### Beispiel 3 : Herstellung von Tobramycin-penta-pantothenat

2 g Tobramycinsulfat und 3,3 g Calciumdipantothenat werden in je 25 ml VE-Wasser gelöst. Danach wurden die beiden Lösungen mit weiteren 25ml VE-Wasser vereinigt. Die entstandene Lösung wurde filtriert und bis zur Trocknung einrotiert, danach wurde der ausgefallene Feststoff in Methanol aufgenommen, zentrifugiert und anschließend bis zur Trocknung einrotiert. Der entstandene Feststoff wurde über Nacht nachgetrocknet.

### Beispiel 4 : Herstellung von Tobramycin-penta-dodecylsulfat

4,6 g Natriumdodecylsulfat wurden in 37,5 ml dest. Wasser gelöst und mit einer zweiten Lösung aus 2,0 g Tobramycinsulfat in 37,5 ml dest. Wasser vereinigt. Die Lösung wird mit 50 ml dest. Wasser verdünnt, wobei sich eine Emulsion bildet, aus der sich dann Ausflockungen absetzen. Der sich abgesetzte Feststoff wird abfiltriert und getrocknet.

### Beispiel 5 : Darstellung von Gentamicin als freie Base durch Umsalzung

1 g Gentamicinsulfat wurden in 25 ml VE-Wasser gelöst und 0,26 g Calciumhydroxid zugegeben. Die milchige Suspension, wurde filtriert und die filtrierte Lösung bis zur Trocknung einrotiert. Der Rückstand im Kolben wurde in Methanol aufgenommen und anschließend bis zur Trocknung einrotiert. Das sulfatfreie Gentamicin lag danach als klare, gelartige, amorphe Substanz im Kolben vor.

### Beispiel 6 : Darstellung von Gentamicin (freie Base) mit einem anionischen Ionenaustauscher

5 g Gentamicinsulfat wurden in 10 ml VE-Wasser gelöst und durch eine Säule mit dem anionischen lonentauscherharz "Dowex 1 x 2, strongly basic, Cl⁻ Form, 50 - 100 Mesh" befüllt. Das Eluat wurde aufgefangen und bis zur Trocknung einrotiert. Das desulfatierte Gentamicin wurde danach getrocknet.

Zuvor wurde das Harz mit VE-Wasser gewaschen, anschließend mit 4 %iger Natronlauge basisch gestellt und danach mit VE-Wasser neutral gewaschen. Das neutral gewaschene Harz wurde anschließend mit Silbernitratlösung auf Chloridfreiheit hin untersucht und noch einmal mit VE-Wasser nachgespült.

### Beispiel 7 : Darstellung von Tobramycin-penta-Dexpanthenol

2,2 g Dexpanthenol wurden in 75 ml Methanol gelöst und anschließend wurde 1g Tobramycin (siehe Beispiel 5 als freie Base) in die Lösung gegeben. Anschließend wurde die Lösung bis zur Trocknung einrotiert.

### Beispiel 8 : . Darstellung von Gentamicin-penta-Dexpanthenol

2 g Gentamicinsulfat wurden in 25 ml VE-Wasser und 0,5 g Calciumhydroxid in die wässrige Lösung gegeben. Es entstand eine milchige Suspension, die bis zur Trocknung einrotiert wurde. Der Rückstand im Kolben wurde in Methanol aufgenommen, filtriert und das Filtrat wurde anschließend bis zur Trocknung einrotiert. Das sulfatfreie Gentamicin lag danach als klare, gelartige, amorphe Substanz vor. 2,2 g Dexpanthenol wurden in 75 ml Methanol gelöst und anschließend wurde 1 g des als freie Base vorliegenden Gentamicins zugegeben und bis zur Trocknung einrotiert.

### Beispiel 9 : Darstellung von Chlorhexidindipantothenat

Calciumdipantothenat wurde mit 0,5 molarer Schwefelsäure versetzt und die wässrige Pantothensäurelösung bis zur Trocknung einrotiert. Der Rückstand im Kolben wurde in Methanol aufgenommen und wieder bis zur Trocknung einrotiert. Anschließend wurde die getrocknete Pantothensäure gewogen, in 75 ml Methanol gelöst und mit Chlorhexidin (freie Base) im Verhältnis Pantothensäure zu Chlorhexidin 2:1 versetzt und bis zur Trocknung einrotiert.

Das Chlorhexidindipantothenat kristallisierte als spröder weißer Feststoff aus. Das stöchiometrische 2:1 Verhältnis konnte mittels HPLC exakt nachgewiesen werden.

### Beispiel 10 : Nachweis von Aminoglykosiden durch HPLC Analyse

Als beispielhaft wird hier der Nachweis von Gentamicinsulfat und Gentamicin-penta-Pantothenat beschrieben. Gentamicinsulfat und Gentamicin-penta-Pantothenat wurden mit einem Ortho-Phthalaldehyd-haltigen Reagenz angefärbt und anschließend mittels HPLC analysiert.

100 µl der Aminoglykosid-haltigen Probe wurden in ein HPLC-Vial gegeben und mit 240 µl Methanol versetzt. Danach wurden 160 µl des Derivatisierungsreagenzes dazugegeben. Die Proben wurden dann 20 min. bei 60° C in einem Trockenschrank erwärmt und anschließend gemessen.

**Tab.1: Nachweis von Gentamicinsulfat mittels HPLC**

| Standard | Volumen Stammlösung [µl] | Volumen VE-Wasser [µl] | Verdünnungsfaktor | Konzentration Gentamicinsulfat [µg/ml] | Peakfläche gesamt [µV*min] | Response [µV*min./µg] |
|---|---|---|---|---|---|---|
| 1 | 25 | 975 | 0,025 | 29,3375 | 79566 | 2712 |
| 2 | 50 | 950 | 0,05 | 58,675 | 163974 | 2795 |
| 3 | 100 | 900 | 0,1 | 117,35 | 321742 | 2742 |
| 4 | 200 | 800 | 0,2 | 234,7 | 629286 | 2681 |

**Tab.2 : Nachweis von Gentamicin-penta-pantothentat mittels HPLC**

| Probenbezeichnung | Peakfläche gesamt [µV*min.] | Konzentration Gentamicin [µg/ml] | Sollwert [µg/ml] | Wiederfindung [%] |
|---|---|---|---|---|
| Blindwert | 0 | -1,98 | - | - |
| Gentamicin-penta-pantothenat | 2444747 | 914,82 | 918,54 | 99,6 |
| Gentamicin-penta-pantothenat | 2202916 | 824,14 | 918,54 | 89,72 |

### Beispiel 11 : Tauchbeschichtung von mikroporösen Titanoberflächen mit Gentamicin-penta-pantothenat

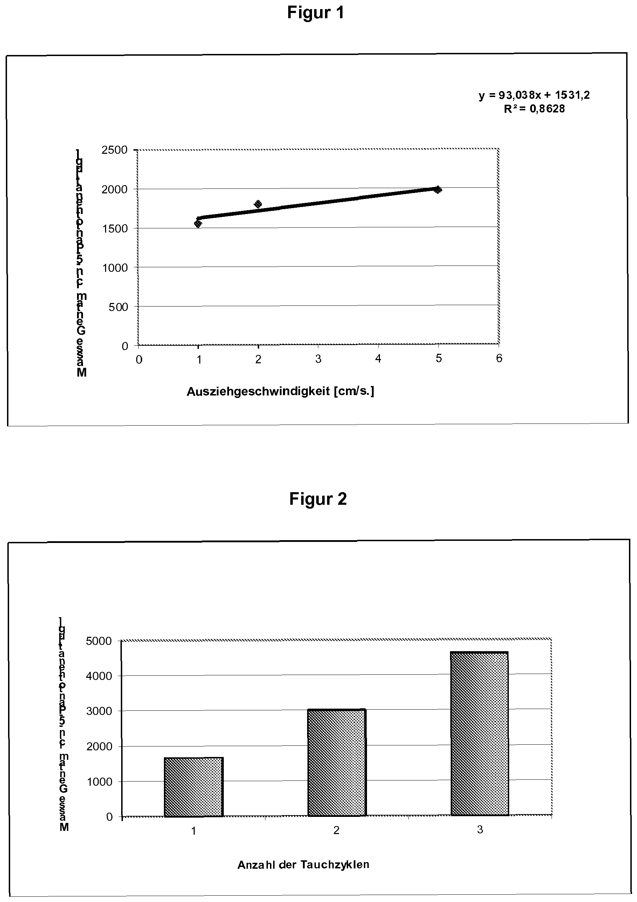

Ethanolische Gentamicin-penta-pantothenat Lösungen mit unterschiedlichen Massenanteilen (10 %, 12,5 %, 15%, 17,5 % und 20 %) wurden hergestellt. Anschließend wurden Probekörper aus Titan mit einer mikroporösen Oberfläche in diese Beschichtungslösungen eingetaucht und nach dem Herausziehen aus der Beschichtungslösung getrocknet. Es konnte nachgewiesen werden, dass durch Erhöhung der Ausziehgeschwindigkeit die Beschichtungsmasse auf den Probenkörpern ansteigt (Fig.1). .Des Weiteren konnte nachgewiesen werden, dass die Beschichtungsmasse auf den Probenkörpern durch Wiederholen der Tauchvorgänge deutlich ansteigt (Fig.2).

### Beispiel 12: Wasserfreie Darstellung von Gentamicin-penta-pantothenat

1,65 g Calciumdipantothenat bei RT in 25 ml DMSO gelöst und 1g Gentamicinsulfat zugefügt. Die leicht trübe Lösung wurde filtriert und anschließend in 75 ml Aceton ausgefällt. Der ausgefallene Feststoff wurde in 25 ml Ethanol aufgenommen und anschließend bis zur Trocknung einrotiert. Der entstandene Feststoff wurde über Nacht nachgetrocknet.

### Beispiel 13 : Sprühbeschichtung mit ethanolischen Gentamicin-penta-pantothenat Lösungen

Gentamicin-penta-pantothenat wird auf aufgeraute Titanoberflächen aufgesprüht. Zu diesem Zweck wurden zylindrische, aufgeraute Titanprüfkörper mit einer nominellen Oberfläche von 3,85 cm² mit unterschiedlichen Sprühzeiten unter Berücksichtigung der nachfolgenden Parameter beschichtet.
Sprühintervall: 15 s
Trockenintervall: 30 s
Sprühlösung: 0,6% iges ethanolisches Gentamicin-penta-pantothenat

**Tab.3 Menge des aufgesprühten Gentamicin-penta-pantothenats**

| Lfd. Nr.: | Sprühzyklen | Sprühzeit (gesamt) [s.] | Masse der Beschichtung [µg] |
|---|---|---|---|
| 1 | 4 | 60 | 2025 |
| 2 | 6 | 90 | 3211 |
| 3 | 8 | 120 | 4904 |
| 4 | 7 | 105 | 3995 |
| 5 | 7 | 105 | 3946 |
| 6 | 7 | 105 | 4002 |

Auf der Oberfläche entstand eine gleichmäßige amorphe Beschichtung, die sehr gut auf Elektronenmikroskop-Aufnahmen zu erkennen ist (Fig.3).

### Beispiel 14 :. Beschichtung einer Endoprothese mit Gentamicin-penta-pantothenat in Kombination mit Daptomycin oder Vancomycin

0,6% iges ethanolisches Gentamicin-penta-pantothenat wird mit einer 0,5%igen ethanolischen Daptomycin (oder Vancomycin-)lösung (1:1;v/v) vermischt und auf eine Hüftgelenksprothese aus medizinischem Edelstahl gleichmäßig aufgesprüht. Die Beschichtung liegt entweder direkt auf der Prothesenoberfläche oder wird auf einer bereits mit z.B. einem biostabilen oder bioabbaubaren Polymeren oder einer mit Hydroxylapatit beschichteten Prothesenoberfläche aufgebracht.

### Beispiel 15 :. Beschichtung von Endoprothesen mit Gentamicin- penta-pantothenat und einer porösen Polysulfontoplayer als Beispiel für eine Beschichtung mit einer zusätzliche Wirkstoffbeschichtung

Das nach Versuch 12 hergestellte Gentamicin-penta-pantothenat wird als 20%ige ethanolische Lösung auf eine Knieprothese pipettiert. Nach Verdampfen des Lösungsmittels wird in einem weiteren Schritt eine Polysulfon / PVP - Lösung (z.B. 0,80 % PS, 0,08 % PVP oder z.B. 0,84 % PS, 0,04 % PVP in Chloroform) im Sprühverfahren aufgetragen. Der Polysulfonsprühlösung kann entweder erneut Gentamicin oder ein weiterer Wirkstoff hinzugefügt werden, z.B. ein weiteres Antibiotikum wie Vancomycin, Fusidinsäure, Rifampicin, etc. oder auch Wirkstoffe wie z.B. Paclitaxel, Rapamycin.

Geeignete Sprühlösungen für eine solche zusätzliche Wirkstoffbeschichtung können sein:
Sprühlösung: 0,58 % PS, 0,22 % Simvastatin, 0,08 % PVP in Chloroform
Sprühlösung : 0,58 % PS, 0,08 % PVP, 0,22 % Paclitaxel in Chloroform
Sprühlösung : 0,62 % PS, 0,22 % Simvastatin, 0,04 % PVP in Chloroform
Sprühlösung : 0,66 % PS, 0,22 % Simvastatin in Chloroform
Sprühlösung : 0,66 % PS, 0,22 % Paclitaxel in Aceton
Sprühlösung : 0,66 % PS, 0,22 % 17-β-Estradiol in Chloroform
Sprühlösung : 0,66 % PS, 0,22 % Trapidil in Chloroform
Sprühlösung : 0,62 % PS, 0,22 % Amikacin, 0,04 % PVP in Chloroform
Sprühlösung : 0,62 % PS, 0,22 % Gentamicin, 0,04 % PVP in Ethanol

### Beispiel 16 : Beschichtung von Endoprothesen mit einem Aminoglykosidpantothenat und einem bioabbaubarem, polymeren Toplayer

Das nach Vorschrift 8 oder 12 hergestellte Aminoglykosidpantothenat wird mittels Pipettierverfahren auf einen Stent aufgetragen.

Anschließend wird äquivalent zu Beispiel 15 eine bioabbaubare polymere Schicht aufgetragen.

Im Folgenden sind Beispiele für geeignete Sprühlösungen genannt:
Sprühlösung: 19,8 mg Polylaktid und 6,6 mg Taxol werden mit Chloroform auf 3 g aufgefüllt
Sprühlösung: 145,2 mg Polylaktid und 48,4 mg Rapamycin werden auf 22 g mit Chloroform aufgefüllt.
Sprühlösung: 22 mg Polylaktid und 22 mg hydrophiler Wirkstoff werden eingewogen und mit Chloroform auf 5 g aufgefüllt.
Sprühlösung: 176 mg Polylaktidglycolid wird eingewogen und mit Chloroform auf 20 g aufgefüllt.
Sprühlösung: 22 mg Polylaktidglycolid und 22mg Kanamycin werden eingewogen und mit Chloroform auf 22 g aufgefüllt.

### Beispiel 17:. Vollflächige Beschichtung von Endoprothesen mit einer Polymer/ Antibiotikum-Pantothenat- Mischung

Polyurethan wird in THF gelöst, so dass man eine 14 %-ige Lösung erhält und mit einem Antibiotikumpantothent der Wahl vermischt, so dass die Lösung einen Wirkstoffanteil von 30 Gew.% enthält. Diese Lösung wird mit THF oder Chloroform auf 10% verdünnt wird und mittels Tauch-, Sprüh- oder Pipettierverfahren auf die Oberfläche der Endoprothese aufgebracht.

### Beispiel 18 : Beschichtung eines Katheters mit Tobramycinpantothenat mittels Pipettierverfahren

Hierzu wird das nach Versuch 7 hergestellte Tobramycinpantothenat als 10%ige ethanolische Lösung mittels Pipettierverfahren auf der Oberfläche des Katheters gleichmäßig verteilt und getrocknet.

### Beispiel 19 : Pipettierbeschichtung von Titanzylindern für Zytotoxizitätstests

Die Mantelflächen von Titanzylindern wurden im Pipettierverfahren mit ethanolischer Gentamicin-penta-pantothenat-Lösung (w=10%) beschichtet.

**Tab. 4 : Pipettierbeschichtung von Titanzylindern mit Gentamicin-penta-pantothenat für den Zytotoxizitätstest**

| Probe | Einwaage | Auswaage | Masse der Beschichtung | | Coating |
|---|---|---|---|---|---|
| Sollbeladung | Mittelwert | Mittelwert | Ist | Soll | |
| [µg/cm²] | [g] | [g] | [mg] | [mg] | [%] |
| 900 | 44,89078 | 44,91764 | 26,86 | 27,14 | 99,0 |
| 800 | 44,79786 | 44,82141 | 23,55 | 24,13 | 97,6 |
| 700 | 44,97982 | 45,00092 | 21,10 | 21,11 | 99,9 |

### Beispiel 20 :. . Pipettierbeschichtung von Titanzylindern für Proliferationstests

Die Mantelflächen der Titanzylinder wurden mittels Pipettierverfahren mit ethanolischer Gentamicin-penta-pantothenat-Lösung (w=1%) beschichtet.

**Tab. 5 : Pipettierbeschichtung von Titanzylindern mit Gentamicin-penta-pantothenat für den Proliferationstest**

| Probe | Einwaage | Auswaage | Masse der Beschichtung | | Coating |
|---|---|---|---|---|---|
| Sollbeladung | Mittelwert | Mittelwert | Ist | Soll | |
| [µg/cm²] | [g] | [g] | [mg] | [mg] | [%] |
| 100 | 44,76058 | 44,76332 | 2,74 | 3,02 | 90,9 |
| 50 | 44,86333 | 44,86472 | 1,39 | 1,51 | 92,0 |
| 20 | 44,87962 | 44,88018 | 0,56 | 0,60 | 92,3 |

### Beispiel 21 : Dosisfindung für Proben zur Beschichtung von Hüftgelenksprothesen mit Gentamicin-penta-panthotenat :

Die Versuche dienen zur Ermittlung der Gentamicinpantothenatbelegungsmenge/cm², die als Untergrenze noch genügend antibakterielle Wirksamkeit zeigt und der höchstmöglichen Belegungsmenge als Obergrenze, die ohne cytotoxische Eigenschaften für die Zellen in der Umgebung des beschichteten Implantats eingesetzt werden kann. Hierzu wurden grob gestrahlte Titanzylinder mittels Pipettierverfahren mit unterschiedlichen Mengen ethanolischer Gentamicin-5-panthothenat-Lösung beschichtet und nach Trocknung die Beschichtungsmasse durch Wägung bestimmt. Die beschichteten Titanröhrchen wurden anschliessend γ-sterilisiert.

Richtwerte für die Obergrenze und Untergrenze der Beschichtungsmenge auf den Prüfkörpern wurden mit Hilfe bereits bekannter Daten zur Cytotoxizität von Gentamicin als reinem Wirkstoff abgeleitet. Hierzu ergab sich als Untergrenze gegenüber teilresistenten *Staphylococcus aureus*-Stämmen eine minimale Hemmkonzentration von 64 µg/mL (Alt et. al, 2004). Die Umrechnung auf Gentamicinpanthotenat/cm² hat damit rechnerisch eine erforderliche minimale Dosis von 28 µg/cm² für die Untergrenze. Die Obergrenze für die Proben wurde aufgrund von Modellrechnungen in Kombination mit experimentell speziell ermittelten Zytotoxizitätsdaten von Gentamicinsulfat festgelegt. Aufgrund der ermittelten Daten wurde eine als unbedenklich eingestufte Maximaldosis von 873 µg/cm² berechnet, allerdings unter der Annahme, dass das Gentamicinpantothenat im ungünstigsten Fall sofort und vollständig freigesetzt wird.

**Tab. 6 : Beschichtungsmengen zur Ermittlung der Dosisunter- und obergrenze auf identischen Titanzylindern mit rauer Oberfläche**

| Probe Nr (je n=3) | Beschichtungsmenge [mg] | Ermittlung der Dosis- |
|---|---|---|
| 1 | 26,9 | Obergrenze |
| 2 | 23,6 | Obergrenze |
| 3 | 21,1 | Obergrenze |
| 4 | 0,6 | Untergrenze |
| 5 | 1,5 | Untergrenze |
| 6 | 3,0 | Untergrenze |

### Beispiel 22 : Versuche zur antimikrobiellen Aktivität

Die Gentamicin-penta-panthotenat Beschichtung der zylindrischen Prüfkörper aus Titan wurde mit je 6 ml Wasser (Extraktionsvolumen wurde entsprechend einer ungünstig hohen Flüssigkeitssäule zwischen Implantat und Knochen von 2 mm bei einer Probenoberfläche von 30 cm² berechnet) entfernt und über 24h bei 30-35°C inkubiert. Anschließend wird die minimale Hemmkonzentration (MIC) für *Staphylococcus aureus* (ATCC 6538) über eine serielle Verdünnungsreihe entsprechend DIN 58940 (Part 7) bestimmt.

**Tab. 7 : Ermittlung der notwendigen Dosierung an Gentamicin-penta-pantothenat für eine wirksame antimikrobiellen Aktivität (Untergrenze)**

| Probe | MIC (Verdünnung der Testlösung) | Errechnete MIC |
|---|---|---|
| Ti-Zylinder, unbeschichtet | Nicht erreicht | - |
| Ti-Zylinder, raue Oberfläche, 20 µg/cm² Gentamicin-penta-pantothenat | 1:2 | 50 µg/mL |
| Ti-Zylinder, raue Oberfläche , 50 µg/cm² | 1:4 | 62,5 µg/mL |
| Gentamicin-penta-pantothenat | | |
| Ti-Zylinder, raue Oberfläche, 100 µg/cm² Gentamicin-penta-pantothenat | 1:16 | 31,25 µg/mL |

| | | |
|---|---|---|
| MIC : Minimale Hemmkonzertation | | |

Es hat sich gezeigt, dass auch bei der niedrigsten Konzentration bereits die aus der Literatur errechnete minimale Hemmkonzentration (MIC) erreicht wird.

### Beispiel 23 : Ermittlung der maximal möglichen Dosierung an Gentamicin-penta-pantothenat zur Vermeidung zytotoxischer Nebenwirkungen (Obergrenze)

Nach 24-stündiger Extraktion der Gentamicin-penta-panthotenat Beschichtung der zylindrischen Prüfkörper aus Titan mit Zellkulturmedium und Inkubation der Extrakte mit L929-Zellen für 68h bei 37°C wurde der Proteingehalt der Proben mittels BCA-Farbtest entsprechend ISO 10993-5 bestimmt. Dies lässt einen Rückschluss auf die Proliferation der Zellen während der Inkubation zu.

**Tab. 8 : Ergebnisse zur Cytotoxicität von Proben mit unterschiedlicher Konzentration an Gentamicin-5-pantothenat**

| Probe | Wachstumsinhibierung [%] | Cytotoxisch nach ISO 10993-5 |
|---|---|---|
| Ti-Zylinder, raue Oberfläche, unbeschichtet | 15 | nein |
| Ti-Zylinder, raue Oberfläche , 700 µg/cm² Gentamicin-penta-pantothenat | 23 | nein |
| Ti-Zylinder, raue Oberfläche, 800 µg/cm² Gentamicin-penta-pantothenat | 18 | nein |
| Ti-Zylinder, raue Oberfläche, 900 µg/cm² Gentamicin-penta-pantothenat | 20 | nein |

### Beispiel 24 : Elutionsversuche am Beispiel von mit Gentamicin-, Gentamicinpantothenat- und Gentamicinpalmitatbeschichteten Titanproben

Hierzu werden die Proben in 2 ml demineralisiertes Wasser gegeben und bei 37° über einen festgesetzten Zeitraum im statischen wässrigen System gehalten. Zu festgelegten Zeiten werden jeweils 50 µL Probenvolumen entnommen. Die Antibiotikumgehaltsbestimmung in der Probe erfolgt mittels Ninhydrin-Farbreaktion. Diese Methode der Gehaltsbestimmung funktioniert mit allen Antibiotika, die Aminogruppen aufweisen, wie am Beispiel der Struktur des Aminoglykosids Gentamicin stellvertretend veranschaulicht:

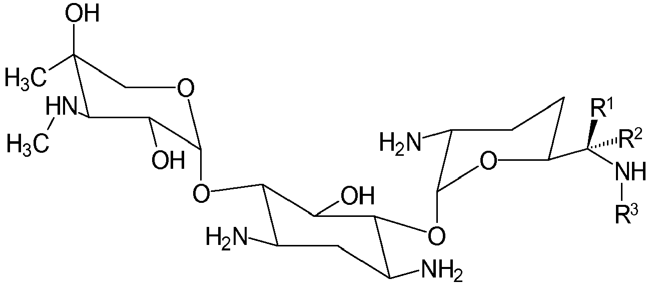

### Beispiel 25 : In vitro Cytotozizität von Calciumpantothenat im Vergleich zu Gentamicinsulfat, Palmitinsäure, Salicylsäure und Benzoesäure

Der cytotoxische Effekt wurde an der Wachstumshemmung der Zellinie L929 nach Inkubationsdauer von 68 - 72 h für 6 verschiedene Konzentrationen nach den zutreffenden gültigen DIN und ISO-Normen getestet. Als Negativkontrolle diente DMEM 10% FCS und die Positivkontrolle war 5% DMSO in DMEM 10% FCS

**Tab. 9 : Vergleichende Aufstellung der Wachstumshemmung der Zelllinie L929 in %**

| Proben (n=3) | | | | | | | | | |
|---|---|---|---|---|---|---|---|---|---|
| Benzoesäure | | Salicylsäure | | Gentamicinsulfat | | Palmitinsäure | | CaPantothenat | |
| c [mg/ml] | GI [%] | c [mg/ml] | GI [%] | c [mg/ml] | GI [%] | c [mg/ml] | GI [%] | c [mg/ml] | GI [%] |
| 3,0 | 77 | 1,3 | 74 | 3,0 | 29 | 0,05 | 30 | 3,0 | 2 |
| 2,1 | 64 | 1,1 | 67 | 2,4 | 22 | 0,04 | 22 | 2,4 | 1 |
| 1,5 | 50 | 0,8 | 54 | 1,8 | 12 | 0,03 | 17 | 1,8 | 7 |
| 0,9 | 35 | 0,5 | 42 | 1,2 | 12 | 0,02 | 8 | 1,2 | 4 |
| 0,6 | 20 | 0,3 | 19 | 0,6 | 8 | 0,01 | 5 | 0,6 | 0 |
| 0,3 | 10 | 0,1 | 16 | 0,3 | 4 | 0,005 | 2 | 0,3 | 0 |
| Pos.C. | 87 | | 86 | | 86 | | 88 | | 89 |
| Neg.C. | 0 | | 0 | | 0 | | 2 | | 5 |
| LM.C. | 0 | | 0 | | 0 | | 0 | | 0 |

| | | | | | | | | | |
|---|---|---|---|---|---|---|---|---|---|
| **a : Solvent control : 0,5%Ethanol mit DMEM 10% FCS, alle anderen DMEM 10% FCS** **Pos.C.** : **positive Kontrolle** **Neg.C.** : **Negative Kontrolle** **S.C. : Lösungsmittelkontrolle** | | | | | | | | | |

### Beispiel 26 : Herstellung von Doxycyclin-di-pantothenat

3,5 g Doxycyclinhydrochloridhemiethanolat (Merck) in 50ml einer 50%igen ethanolischen Lösung in VE-Wasser und 3,3 g Calciumdipantothenat, in 35 ml VE-Wasser gelöst, wurden zueinander gegeben und mit weiteren 35 ml Wasser vereinigt. Die Lösung wurde filtriert und im Rotationsverdampfer bei 50° C bis zur Trocknung einrotiert und der ausgefallene Feststoff in Methanol aufgenommen. Die methanolische Suspension wurde anschließend zentrifugiert und das Sediment über Nacht nachgetrocknet.

### Beispiel 27 : Herstellung von Tetracyclin-mono-pantothenat

3,5 g Tetracyclinhydrochlorid (Merck) in 50ml einer 50%igen ethanolischen Lösung in VE-Wasser und 1,7 g Calciumdipantothenat, in 35 ml VE-Wasser gelöst, wurden zueinander gegeben und mit weiteren 35 ml Wasser vereinigt. Die Lösung wurde filtriert und im Rotationsverdampfer bei 50° C bis zur Trocknung einrotiert und der ausgefallene Feststoff in Methanol aufgenommen. Die methanolische Suspension wurde anschließend zentrifugiert und das Sediment über Nacht nachgetrocknet.

### Beispiel 28 : Herstellung von Minocyclin-mono-pantothenat

3,3 g Minocyclinhydrochlorid (Sigma Aldrich) in 50ml einer 50%igen ethanolischen Lösung in VE-Wasser und 1,7 g Calciumdipantothenat, in 35 ml VE-Wasser gelöst, wurden zueinander gegeben und mit weiteren 35 ml Wasser vereinigt. Die Lösung wurde filtriert und im Rotationsverdampfer bei 50° C bis zur Trocknung einrotiert und der ausgefallene Feststoff in Methanol aufgenommen. Die methanolische Suspension wurde anschließend zentrifugiert und das Sediment über Nacht nachgetrocknet.

### Beispiel 29 : Herstellung von Clindamycin-mono-pantothenat

3,3 g Clindamycinhydrochlorid (AppliChem) in 50ml einer 50%igen ethanolischen Lösung in VE-Wasser und 1,7 g Calciumdipantothenat, in 35 ml VE-Wasser gelöst, wurden zueinander gegeben und mit weiteren 35 ml Wasser vereinigt. Die Lösung wurde filtriert und im Rotationsverdampfer bei 50° C bis zur Trocknung einrotiert und der ausgefallene Feststoff in Methanol aufgenommen. Die methanolische Suspension wurde anschließend zentrifugiert und das Sediment über Nacht nachgetrocknet.

### Beispiel 30: Wasserfreie Darstellung von Tetracyclin-mono-pantothenat

1,65 g Calciumdipantothenat bei RT in 25 ml DMSO gelöst und 3 g Tetracyclinhydrochlorid zugefügt. Die leicht trübe Lösung wurde filtriert und anschließend in 75 ml Aceton ausgefällt. Der ausgefallene Feststoff wurde in 25 ml Ethanol aufgenommen und anschließend bis zur Trocknung einrotiert. Der entstandene Feststoff wurde über Nacht nachgetrocknet.

### Beispiel 31 : Darstellung von Tetracyclin als freie Base durch Umsalzung

1 g Tetracyclinhydrochlorid wurden in 25 ml VE-Wasser gelöst und 0,26 g Calciumhydroxid zugegeben. Die milchige Suspension wurde bis zur Trockne einrotiert. Der Rückstand im Kolben wurde in Ethanol aufgenommen, die resultierende Suspension filtriert und das Filtrat anschließend bis zur Trockne eingeengt. Das chloridfreie Tetracyclin lag danach als gelber, amorpher Feststoff im Kolben vor.

### Beispiel 32 : Darstellung von Doxycyclin als freie Base durch Umsalzung

1 g Doxycyclinhydrochloridhemiethanolat wurden in 25 ml VE-Wasser gelöst und 0,25 g Calciumhydroxid zugegeben. Die milchige Suspension wurde bis zur Trockne einrotiert. Der Rückstand im Kolben wurde in Ethanol aufgenommen, die resultierende Suspension filtriert und das Filtrat anschließend bis zur Trockne eingeengt. Das chloridfreie Doxycyclin lag danach als gelber, amorpher Feststoff im Kolben vor.

### Beispiel 33 : Darstellung von Tetracyclin-mono-Dexpanthenol

2,2 g Dexpanthenol wurden in 75 ml Methanol gelöst und anschließend wurde 1g Tetracyclin (siehe Beispiel 31 als freie Base) in die Lösung gegeben. Anschließend wurde die Lösung bis zur Trocknung einrotiert.

### Beispiel 34 : . Darstellung von Doxycyclin-mono-Dexpanthenol

2,2 g Dexpanthenol wurden in 75 ml Methanol gelöst und anschließend wurde 1 g des als freie Base vorliegenden chloridfreien Doxycyclin (siehe Beispiel 32) zugegeben und bis zur Trocknung einrotiert.

### Beispiel 39 : Tauchbeschichtung eines mikroporösen Verankerungsstiftes mit Tetracyclin-mono-pantothenat

Ethanolische Tetracyclin-mono-pantothenat-Lösungen mit unterschiedlichen Massenanteilen (10 %, 12,5 %, 15%, 17,5 % und 20 %) wurden hergestellt. Es konnte nachgewiesen werden, dass durch Erhöhung der Ausziehgeschwindigkeit die Beschichtungsmasse auf den Probenkörpern ansteigt. Des Weiteren konnte nachgewiesen werden, dass die Beschichtungsmasse auf den Probenkörpern durch Wiederholen der Tauchvorgänge deutlich ansteigt. Ferner konnte nachgewiesen werden, dass durch Erhöhung der Konzentration der Tetracyclin-mono-pantothenat Lösungen die Schichtdicken pro Tauchvorgang proportional ansteigt.

### Beispiel 40 : Tauchbeschichtung einer mikroporösen Zahnprothese mit Clindamycin-mono-pantothenat

Ethanolische Clindamycin-mono-pantothenat mit unterschiedlichen Massenanteilen (10 %, 12,5 %, 15%, 17,5 % und 20 %) wurden hergestellt. Es konnte nachgewiesen werden, dass durch Erhöhung der Ausziehgeschwindigkeit die Beschichtungsmasse auf den Probenkörpern ansteigt. Des Weiteren konnte nachgewiesen werden, dass die Beschichtungsmasse auf den Probenkörpern durch Wiederholen der Tauchvorgänge deutlich ansteigt. Ferner konnte nachgewiesen werden, dass durch Erhöhung der Konzentration der Clindamycin-mono-pantothenat-Lösungen die Schichtdicken pro Tauchvorgang proportional ansteigt.

### Beispiel 41 : Beschichtung eines Fingergelenkimplamtats mit Doxycyclin-di-pantothenat mittels Pipettierverfahren

Hierzu wird das nach Versuch 26 hergestellte Doxycyclin-di-pantothenat als 10%ige ethanolische Lösung mittels Pipettierverfahren auf der Oberfläche des Katheters gleichmäßig verteilt.

### Beispiel 42 : Beschichtung eines Fingergelenkimplamtats mit Tetracyclin-mono-pantothenat mittels Pipettierverfahren

Hierzu wird das nach Versuch 27 hergestellte Tetracyclin-mono-pantothenat als 10%ige ethanolische Lösung mittels Pipettierverfahren auf der Oberfläche des Katheters gleichmäßig verteilt.

### Beispiel 43 : Sprühbeschichtung mit ethanolischen Tetracyclin-mono-Dexpanthenol-Lösungen

Tetracyclin-mono-Dexpanthenol wird auf aufgeraute Titanoberflächen eines Schultergelenkimplantates aufgesprüht. Zu diesem Zweck wurden die Schultergelenkimplantate mit unterschiedlichen Sprühzeiten unter Berücksichtigung der nachfolgenden Parameter beschichtet.
Sprühintervall: 15 s
Trockenintervall: 30 s
Sprühlösung: 0,4% iges ethanolisches Tetracyclin-mono-Dexpanthenol

**Tab. 10**

| Lfd. Nr.: | Sprühzyklen | Sprühzeit (gesamt) [s.] | Coating Masse [µg] |
|---|---|---|---|
| 1 | 4 | 60 | 1025 |
| 2 | 6 | 90 | 1644 |
| 3 | 8 | 120 | 2302 |
| 4 | 7 | 105 | 2001 |
| 5 | 7 | 105 | 1977 |
| 6 | 7 | 105 | 2012 |

### Beispiel 44 : Sprühbeschichtung mit ethanolischen Doxycyclin-di-Dexpanthenol-Lösungen

Doxycyclin-di-Dexpanthenol wird auf aufgeraute Titanoberflächen eines Schultergelenkimplantates aufgesprüht. Zu diesem Zweck wurden die Schultergelenkimplantate mit unterschiedlichen Sprühzeiten unter Berücksichtigung der nachfolgenden Parameter beschichtet.
Sprühintervall: 15 s
Trockenintervall: 30 s
Sprühlösung: 0,4% iges ethanolisches Doxycyclin-mono-Dexpanthenol

**Tab. 11**

| Lfd. Nr.: | Sprühzyklen | Sprühzeit (gesamt) [s.] | Coating Masse [µg] |
|---|---|---|---|
| 1 | 4 | 60 | 1618 |
| 2 | 6 | 90 | 2445 |
| 3 | 8 | 120 | 3255 |
| 4 | 7 | 105 | 3002 |
| 5 | 7 | 105 | 3021 |
| 6 | 7 | 105 | 3001 |

### Beispiel 45 : Sprühbeschichtung mit ethanolischen Minocyclin-mono-pantothenat-Lösungen

Minocyclin-mono-pantothenat wird auf aufgeraute Titanoberflächen eines Schultergelenkimplantates aufgesprüht. Zu diesem Zweck wurden die Schultergelenkimplantate mit unterschiedlichen Sprühzeiten unter Berücksichtigung der nachfolgenden Parameter beschichtet.
Sprühintervall: 15 s
Trockenintervall: 30 s
Sprühlösung: 0,4% iges ethanolisches Minocyclin-mono-pantothenat

**Tab. 12**

| Lfd. Nr.: | Sprühzyklen | Sprühzeit (gesamt) [s.] | Coating Masse [µg] |
|---|---|---|---|
| 1 | 4 | 60 | 1467 |
| 2 | 6 | 90 | 2036 |
| 3 | 8 | 120 | 2949 |
| 4 | 7 | 105 | 2455 |
| 5 | 7 | 105 | 2611 |
| 6 | 7 | 105 | 2487 |

## Patentansprüche

1. Endoprothese, charakterisiert durch eine Wirkstoffbeschichtung bestehend aus mindestens einem Antibiotikum und mindestens einer Substanz der folgenden Formeln

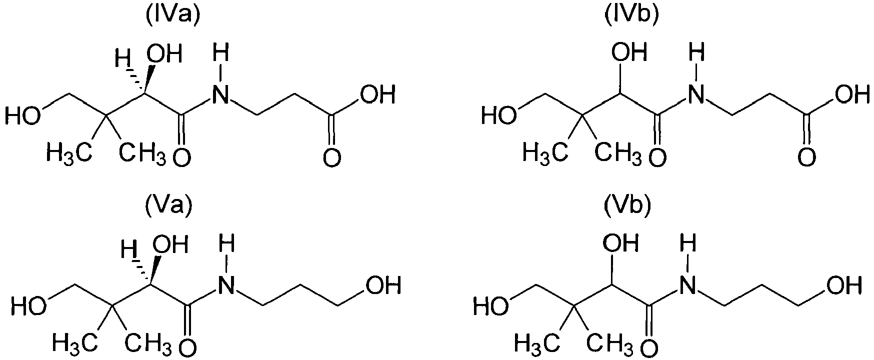

2. Endoprothese nach Anspruch 1, wobei das Antibiotikum ausgewählt wird aus der Gruppe der Aminoglykosid-Antibiotika.

3. Endoprothese nach Anspruch 2, wobei das Aminoglykosid-Antibiotikum ausgewählt wird aus der Gruppe umfassend Streptomycin, Neomycin, Framycetin, Paromomycin, Ribostamycin, Kanamycin, Amikacin, Arbekacin, Bekanamycin, Dibekacin, Tobramycin, Spectinomycin, Hygromycin B, Paromomycinsulfat, Gentamicin, Netilmicin, Sisomicin, Isepamicin, Verdamicin, Astromicin, Apramycin, Ansamycine, Rifampicin und Geneticin.

4. Endoprothese nach einem der vorangegangenen Ansprüche, wobei die Endoprothese ausgewählt ist aus der Gruppe umfassend Zahnimplantate, Herzschrittmacher, Stents, Gefäßprothesen, Hirnschrittmacher, Kunstherzen, Portkatheter, orthopädische Implantate, Sehimplantate, Retina, Glaskörper, Cornea, Schädelrekonstruktionen, Knochenersatz, Penisprothesen, Schließmuskelprothesen, Cochleaimplantate, Katheter, Blasenkatheter, Beatmungsschläuche, Venenkatheter oder Kanülen.

5. Endoprothese nach Anspruch 4, wobei das orthopädische Implantat ausgewählt ist aus der Gruppe umfassend: Wirbelsäulenimplantate, Hüftgelenkimplantate, Pedikelschraube, Knochenkeil, Knochenschraube, Schultergelenkimplantate, Ellenbogengelenksimplantate, Zwischenwirbelimplantate, Fingergelenkimplantate, Fußgelenkimplantate, Zehgelenkimplantate, Kniegelenkimplantate, Sprunggelenkimplantate, Handgelenkimplantate, Implantate für die Fusion von Knochen, Radiuskopf-Implantate, Verankerungsstifte von Implantaten oder für Implantate, Implantate für die Schädeldecke, Korrekturkeile, Winkelimplantate, Implantate für Winkelosteotomien (Tibiakopf), Mittelfußoperationen, Rückfußoperationen.

6. Endoprothese nach einem der vorangegangenen Ansprüche, wobei die Endoprothese ferner eine Basisbeschichtung und/oder ein Toplayer aus Polymeren umfasst.

7. Endoprothese nach Anspruch 6, wobei die Basisbeschichtung und/oder das Toplayer aus bioabbaubaren Polymeren bestehen/besteht.

8. Verfahren zur Beschichtung einer Endoprothese umfassend die folgenden Schritte:
a) Bereitstellung einer unbeschichteten Endoprothese,
b) Bereitstellen einer Beschichtungslösung bestehend aus mindestens ein Antibiotikum und eine Substanz der Formel (IVa) und/oder (IVb) und/oder (Va) und/oder (Vb) in mindestens einem Lösungsmittel,
c) Aufbringen der Beschichtungslösung mittels Sprühen, Tauchen, Streichen, Pinseln, Pipettieren, Gasphasenabscheidung oder Spritzen.

## Claims

1. Endoprosthesis **characterized by** an active agent coating comprising or consisting of at least one antibiotic agent and at least one substance of the following formulas

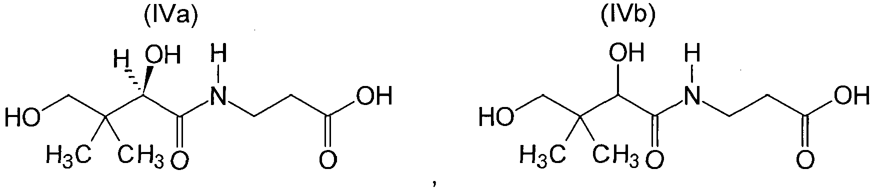

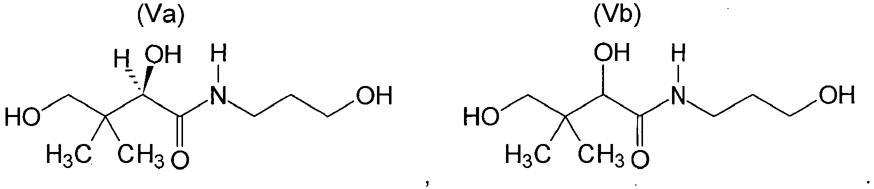

2. Endoprosthesis according to claim 1, wherein the antibiotic agent is selected from the group of amino glycoside antibiotics.

3. Endoprosthesis according to claim 2, wherein the amino glycoside antibiotic is selected from the group comprising streptomycin, neomycin, framycetin, paromomycin, ribostamycin, kanamycin, amikacin, arbekacin, bekanamycin, dibekacin, tobramycin, spectinomycin, hygromycin B, paromomycin sulfate, gentamicin, netilmicin, sisomicin, isepamicin, verdamicin, astromicin, apramycin, ansamycine, rifampicin and geneticin.

4. Endoprosthesis according to any one of the previous claims, wherein the endoprosthesis is selected from the group comprising dental implants, pacemakers, stents, vascular grafts, brain pacemakers, artificial hearts, port catheter, orthopedic implants, visual implant, retina, vitreous body, cornea, skull reconstruction, bone replacement, penile prostheses, sphincter prostheses, cochlear implants, catheters, urinary catheters, breathing hoses, venous catheters or cannulas.

5. Endoprosthesis according to claim 4, wherein the orthopedic implant is selected from the group comprising spinal implants, hip joint implants, pedicle screw, wedge of bone, bone screw, shoulder joint implants, elbow implants, intervertebral implants, finger joint implants, ankle implants, toe joints implants, knee implants, subtalar joint implants, wrist implants, implants for fusion of bone, radial head implants, anchoring pins of implants or for implants, implants for the skull, correction wedges, angle implants, implants for osteotomies (high tibial osteotomy), metatarsal surgery, hindfoot surgery.

6. Endoprosthesis according to any one of the previous claims, wherein the endoprosthesis further comprises a base coat and/or a top layer of polymers.

7. Endoprosthesis according to claim 6, wherein the base coat and/or the top layer consist(s) of biodegradable polymers.

8. Method for coating of an endoprosthesis comprising the following steps:
a) providing an uncoated endoprosthesis,
b) providing a coating solution containing at least one antibiotic and a substance of formula (IVa) and/or (IVb) and/or (Va) and/or (Vb) in at least one solvent,
c) applying the coating solution by spraying, dipping, brushing, painting, pipetting, vapor deposition or spattering.

## Revendications

1. Endoprothèse **caractérisée par** un revêtement d'agent actif comprenant ou constitué d'au moins un agent antibiotique et d'au moins une substance ayant l'une des formules suivantes

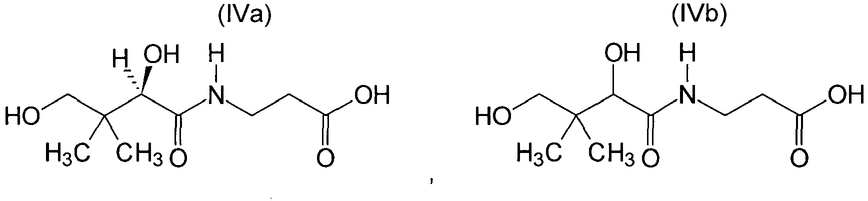

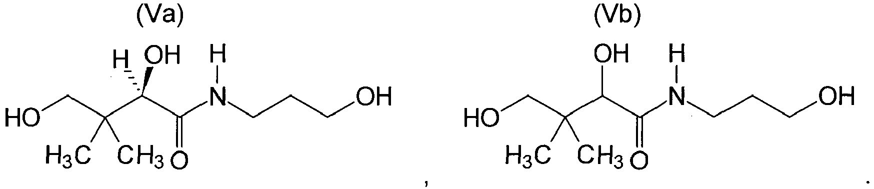

2. Endoprothèse selon la revendication 1, dans laquelle l'agent antibiotique est sélectionné parmi les antibiotiques aminoglycosides.

3. Endoprothèse selon la revendication 2, dans laquelle l'antibiotique aminoglycoside est sélectionné parmi le groupe comprenant la streptomycine, la néomycine, la framycétine, la paromomycine, la ribostamycine, la kanamycine, l'amikacine, l'arbékacine, la békanamycine, la dibékacine, la tobramycine, la spectinomycine, l'hygromycine B, le sulfate de paromomycine, la gentamicine, la nétilmicine, la sisomicine, l'isépamicine, la verdamicine, l'astromicine, l'apramycine, l'ansamycine, la rifampicine et la généticine.

4. Endoprothèse selon l'une quelconque des revendications précédentes, dans laquelle l'endoprothèse est sélectionnée parmi le groupe comprenant les implants dentaires, les stimulateurs cardiaques, les stents, les greffes vasculaires, les stimulateurs cérébraux, les coeurs artificiels, le cathéter à chambre implantable, les implants orthopédiques, l'implant visuel, de la rétine, du corps vitreux, de la cornée, la reconstruction du crâne, le remplacement osseux, les prothèses péniennes, les prothèses sphinctériennes, les implants cochléaires, les cathéters, les cathéters urinaires, les tuyaux respiratoires, les cathéters veineux ou les canules.

5. Endoprothèse selon la revendication 4, dans laquelle l'implant orthopédique est sélectionné parmi le groupe comprenant les implants spinaux, les implants pour l'articulation de la hanche, la vis pédiculaire, le coin osseux, la vis osseuse, les implants pour l'articulation de l'épaule, les implants du coude, les implants intervertébraux, les implants pour l'articulation du doigt, les implants de la cheville, les implants pour les articulations de l'orteil, les implants du genou, les implants pour l'articulation sous-astragalienne, les implants du poignet, les implants pour la fusion osseuse, les implants de tête radiale, les broches d'ancrage des implants ou pour les implants, les implants pour le crâne, les coins de correction, les implants d'angle, les implants pour les ostéotomies (ostéotomie tibiale haute), l'opération du métatarse, l'opération de l'arrière-pied.

6. Endoprothèse selon l'une quelconque des revendications précédentes, dans laquelle l'endoprothèse comprend en outre une couche de base et / ou une couche supérieure de polymères.

7. Endoprothèse selon la revendication 6, dans laquelle la couche de base et / ou la couche supérieure sont constituées de polymères biodégradables.

8. Procédé de revêtement d'une endoprothèse comprenant les étapes suivantes :
a) fournir une endoprothèse sans revêtement,
b) fournir une solution de revêtement contenant au moins un antibiotique et une substance de formule (IVa) et / ou (IVb) et / ou (Va) et / ou (Vb) dans au moins un solvant,
c) appliquer la solution de revêtement par pulvérisation, immersion, brossage, peinture, pipetage, dépôt en phase vapeur ou par éclaboussement.
